# EUROPEAN PATENT APPLICATION

(11) **EP 1 835 289 A1**
(43) Date of publication of application: **19.09.2007**
(21) Application number: 06111054.0
(22) Date of filing: 13.03.2006
(51) Int. Cl.: G01N 33/74

(54) **Means and methods for predicting cardiovascular complications in subjects suffering from osteoporosis**

(71) Applicant: Roche Diagnostics GmbH, 68305 Mannheim (DE); F. Hoffmann-la Roche AG, 4070 Basle (CH)
(72) Inventor: Spanuth, Eberhard., Dr, 69221 Dossenheim (DE)
(74) Representative: Dick, Alexander

(57) **Abstract**

The present invention relates to a method of predicting whether a subject suffering from a bone mineral disorder (e.g. osteoporosis) is at risk of developing cardiovascular complications comprising determining the amount of a natriuretic peptide in a sample of said subject and predicting whether the said subject is at risk of developing cardiovascular complications by comparing the amount to a reference amount. Moreover, the method relates to devices and kits for carrying out said method. The preferred natriuretic peptide marker is proBNP.

## Description

The present invention relates to a method of predicting whether a subject suffering from a bone mineral disorder is at risk of developing cardiovascular complications comprising determining the amount of a natriuretic peptide in a sample of said subject and predicting whether the said subject is at risk of developing cardiovascular complications by comparing the amount to a reference amount. Moreover, the method relates to devices and kits for carrying out said method.

An aim of modem medicine is to provide personalized or individualized treatment regimens. Those are treatment regimens which take into account a patient's individual needs or risks. A particularly important risk of developing cardiovascular complications is the presence of a cardiovascular impairment, such as atherosclerosis, or an unrecognized cardiovascular disease, such as coronary heart disease or ventricular dysfunction. The prevalence of cardiovascular complications is elevated in subjects suffering from cardiovascular impairment or cardiovascular diseases. Cardiovascular diseases are the leading cause of morbidity and mortality in the Western hemisphere. A cardiovascular disease can remain asymptomatic for long periods of time. Therefore, reliable diagnosis of the presence of a cardiovascular disease is more difficult and error-prone than generally believed and comprises clinical evaluation, electrocardiogram, chest X-ray, radionuclide ventriculography, cardiac magneticresonance imaging and other imaging techniques, laboratory tests, echocardiography and invasive investigations, such as cardiac catheterization (Task Force Report: Guidelines for the diagnosis and treatment of chronic heart failure. Eur Heart J 2001; 22: 1527-1560)

It has been speculated that there might be a pathophysiological relationship between osteoporosis and the risk of developing cardiovascular complications independent of the known risk factors for cardiovascular complications such as age, diabetes, dislipidemia or hypertension (Tankó 2005, J. Bone Miner. Res. 20: 1912-1920). However, a specific pathophysiological mechanism has not yet been revealed. In light of this lack of a specific pathophysiological mechanism, risk stratification for cardiovascular complications in postmenopausal females is an emerging issue since said females often develop bone mineral disorders or even osteoporosis. Markers for inflammation, such as CRP or homocysteine have been suggested to be indicative for the bone density; however, theses markers could not be associated with the occurrence of cardiovascular complications. (Ganesan 2005, J. Natl. Med. Assoc. 97: 329-333). For cardiovascular risk stratification in postmenopausal osteoporotic females no biochemical marker is available. Natriuretic Peptides are known to be molecular markers for cardiovascular disease progression. WO 02/083913 discloses that the brain natriuretic peptide (BNP) and variants thereof may be used to predict near-term mortality and morbidity in heart failure patients. However, BNP is suitable as a biomarker according to the prior art only in patients suffering already from heart diseases.

Therefore, the current methods for assessing the risk of developing cardiovascular complications usually require evaluation of a variety of clinical parameters as well as various diagnostic measurements using operative and invasive techniques. Furthermore, a reliable diagnosis usually requires a specialized cardiologist.

Therefore, there is a clear long-standing need for means and methods allowing prediction of whether a subject suffering from a bone mineral disease or disorder is at risk of developing cardiovascular complications in addition. The said means and methods shall allow a reliable efficient prediction and shall avoid the drawbacks of the current techniques.

Thus, the technical problem underlying the present invention must be seen as the provision of means and methods for complying with the aforementioned needs.
The technical problem is solved by the embodiments characterized in the claims and herein below.

Accordingly, the present invention relates to a method of predicting whether a subject suffering from bone mineral disease or disorders is at risk of developing cardiovascular complications comprising:
(a) determining the amount of a natriuretic peptide in a sample of said subject; and
(b) predicting whether the subject is at risk of developing cardiovascular complications by comparing the amount determined in step (a) to a reference amount.

The method of the present invention is, preferably, an in vitro method. The method may comprise further steps such as steps for sample pre-treatment or steps for further evaluation of the results obtained by the comparison in step (b). The method may be carried out manually or assisted by automation. Preferably, step (a) and/or (b) may in total or in part be assisted by automation, e.g., by a suitable robotic and sensory equipment for the determination in step (a) or a computer-implemented prediction in step (b).

The term "predicting" as used herein means assessing the probability according to which a subject will suffer from a cardiovascular complication within a defined time window (predictive window) in the future. The predictive window is an interval in which the subject will develop cardiovascular complications according to the predicted probability. The predictive window may be the entire remaining lifespan of the subject upon analysis by the method of the present invention. Preferably, however, the predictive window is an interval of one, two, three, four, five or ten years after the subject has been subject to the method of the present invention (more preferably and precisely, after the sample to be analyzed by the method of the present invention has been obtained). As will be understood by those skilled in the art, such an assessment is usually not intended to be correct for 100% of the subjects to be analyzed. The term, however, requires that the assessment will be valid (i.e. the subject will indeed develop cardiovascular complications) for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Predicting according to the present invention also includes confirmation and classification of the cardiovascular complications which will be developed by the analyzed subject. In this context, confirmation relates to strengthening or substantiating a prediction by analyzing or monitoring other clinical parameters or markers in addition to the natriuretic peptides referred to in accordance with the present invention. Classification relates to further define the cardiovascular complication as discussed elsewhere in this specification.

The term "subject" as used herein relates to animals, preferably mammals, and humans regardless of their age, gender or subpopulation. However, it is envisaged by the present invention that the subject shall suffer from a bone mineral disorder as specified elsewhere in this specification. Moreover, the subject referred to in accordance with the present invention is, preferably, a subject which has not yet developed symptoms associated with cardiovascular diseases, i.e. a subject which is symptomatically healthy with respect to cardiovascular diseases or complications. Whether a subject has ever developed such symptoms may be derived from its medical history or from persisting clinical signs for cardiovascular disease. Preferably, the subject is a female and, most preferably, said female is in a postmenopausal stage of its development.

The term "bone mineral disorder" as used in accordance with the present invention refers to disorders or diseases which result in a reduced bone mineral density (BMD). The bone mineral density can be determined by various techniques well known in the art. Dual Energy X-ray Absorptiometry (DEXA) is the most common method used to measure bone density and requires no additional pre-treatment of the subject. The patient, e.g., simply lies on a padded table during the scan of a particular part of the body such as the lower spine and hip. The test period is short - only several minutes. A radiologist, subsequently, reads and compares the results to normal values and prepares a concise report for the referring physician. Another method for determining bone mineral density is Quantitative Computed Tomography (QCT). This method is also extremely accurate in measuring bone density in the spine or hip. Thus, the reduction of the bone mineral density referred to above shall be a statistically significant reduction. Preferably, the bone mineral disorder referred to in accordance with the present invention is a bone mineral disease and, more preferably, osteoporosis. Osteoporosis is characterized by a loss of bone mass caused by a deficiency in calcium, vitamin D, magnesium and other vitamins and minerals. A diagnostic criteria of osteoporosis is a BMD value which is less than 2.5 times the standard deviation of the age dependent average of the BMD. It is to be understood that the bone mineral density may vary between different subjects. The most widely accepted clinical criteria are based on T- and Z-scores, derived statistically from an age-dependent BMD reference range that includes healthy young adults.

The term "cardiovascular complication" as used herein refers to any chronic disorder of the cardiovascular system or any acute cardiovascular event. Preferably, a chronic disorder of the cardiovascular system as used herein encompasses coronary heart diseases, stable angina pectoris (SAP) or chronic heart failure (CHF). Acute cardiovascular events are, preferably, acute coronary syndromes (ACS). ACS patients can show unstable angina pectoris (UAP) or myocardial infarction (MI). MI can be an ST-elevation MI (STEMI) or a non-ST-elevated MI (NSTEMI). The occurring of an MI can be followed by a left ventricular dysfunction (LVD).

Further preferred cardiovascular complications encompass cardiovascular diseases such as thrombosis, preferably arterial thrombosis, or diseases causing blood vessel calcification, preferably atherosclerosis.

Furthermore, individuals suffering from a cardiovascular complication can be individuals suffering from stroke. The term "stroke" relates to any cerebro-vascular event in which blood flow to small or large regions of the brain is interrupted, e.g due to haemorrhage into the brain or a thrombosis of a cerebral artery. A stroke may result in temporary loss of consciousness or paralysis. In this case, the stroke is termed "apoplectic stroke".

The individuals may show clinical symptoms (e.g. dyspnea, chest pain) and they may be asymptomatic. Although the present invention is particularly advantageous to identify risk patients showing no symptoms of a preexisting cardiovascular disease, risk, or complication, it is also useful in other settings, e.g to confirm or monitor the risk status of the patient showing symptoms of a cardiovascular disease.

Determining the amount of a natriuretic peptide according to the present invention relates to measuring the amount or concentration, preferably semi-quantitatively or quantitatively. Measuring can be done directly or indirectly. Direct measuring relates to measuring the amount or concentration of the natriuretic peptide based on a signal which is obtained from the natriuretic peptide itself and the intensity of which directly correlates with the number of molecules of the peptide present in the sample. Such a signal - sometimes referred to herein as intensity signal -may be obtained, e.g., by measuring an intensity value of a specific physical or chemical property of the natriuretic peptide. Indirect measuring includes measuring of a signal obtained from a secondary component (i.e. a component not being the natriuretic peptide itself) or a biological read out system, e.g., measurable cellular responses, ligands, labels, or enzymatic reaction products.

In accordance with the present invention, determining the amount of the natriuretic peptide can be achieved by all known means for determining the amount of a peptide in a sample. Said means comprise immunoassay devices and methods which may utilize labled molecules in various sandwich, competition, or other assay formats. Said assays will develop a signal which is indicative for the presence or absence of the natriuretic peptide. Moreover, the signal strength can, preferably, be correlated directly or indirectly (e.g. reverse- proportional) to the amount of polypeptide present in a sample. Further suitable methods comprise measuring a physical or chemical property specific for the natriuretic peptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

In a preferred embodiment, the method for determining the amount of a natriuretic peptide comprises the steps of (a) contacting a cell capable of eliciting a cellular response the intensity of which is indicative of the amount of the peptide with the peptide for an adequate period of time, (b) measuring the cellular response.
For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide.

In another preferred embodiment, the method for determining the amount of a natriuretic peptide comprises the step of measuring a specific intensity signal obtainable from the natriuretic peptide in the sample.
As described above, such a signal may be the signal intensity observed at an m/z variable specific for the natriuretic peptide observed in mass spectra or a NMR spectrum specific for the natriuretic peptide.

In another preferred embodiment, the method for determining the amount of a natriuretic peptide comprises the steps of (a) contacting the peptide with a specific ligand, (b) (optionally) removing non-bound ligand, (c) measuring the amount of bound ligand.
The bound ligand will generate an intensity signal. Binding according to the present invention includes both covalent and non-covalent binding. A ligand according to the present invention can be any compound, e.g., a peptide, polypeptide, nucleic acid, or small molecule, binding to the natriuretic peptide described herein. Preferred ligands include antibodies, nucleic acids, peptides or polypeptides such as receptors for the natriuretic peptides and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the natriuretic peptide. "Specific binding" according to the present invention means that the ligand or agent should not bind substantially to ("cross-react" with) another peptide, polypeptide or substance present in the sample to be analyzed. Preferably, the specifically bound natriuretic peptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Suitable methods are described in the following.
First, binding of a ligand may be measured directly, e.g. by NMR or surface plasmon resonance.
Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the ligand/natriuretic peptide complex or the ligand which was bound by the natriuretic peptide may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

In another preferred embodiment, the method for determining the amount of a natriuretic peptide comprises (a) contacting a solid support comprising a ligand for the natriuretic peptide as specified above with a sample comprising the natriuretic peptide and (b) measuring the amount of natriuretic peptide which is bound to the support.
The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of the natriuretic peptides, the relative amount or concentration of the natriuretic peptides as well as any value or parameter which correlates thereto. Such values or parameters comprise intensity signal values from all specific physical or chemical properties obtained from the natriuretic peptides by direct measurements, e.g., intensity values in mass spectra or NMR spectra. Moreover, encompassed are all values or parameters which are obtained by indirect measurements specified elsewhere in this description, e.g., expression levels determined from biological read out systems in response to the natriuretic peptide or intensity signals obtained from specifically bound ligands. It is to be understood that values correlating to the aforementioned amounts or parameters can also be obtained by all standard mathematical operations.

The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, R.O. (1996). New insights into the cardiac natriuretic peptides. Circulation 93: 1946-1950).

ANP-type peptides comprise pre-proANP, proANP, NT-proANP, and ANP.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith MW, Espiner EA, Yandle TG, Charles CJ, Richards AM. Delayed metabolism of human brain natriuretic peptide reflects resistance to neutral endopeptidase. J Endocrinol. 2000; 167: 239-46.).

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller T, Gegenhuber A, et al., Clin Chem Lab Med 2004; 42: 942-4, supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73.). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP as referred to in accordance with the present invention is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913, Bonow 1996, New Insights into the cardiac natriuretic peptides. Circulation 93: 1946-1950. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein.

The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999. Development of a novel, N-Terminal-proBNP (NT-proBNP) assay with a low detection limit. Scand J Clin Invest 59:177-181), Yeo et al. (Yeo 2003. Multicenter evaluation of the Roche NT-proBNP assay and comparison to the Biosite Triage assay. Clinica Chimica Acta 338:107-115), and in Example 1, below.

Variants also include post-translationally modified peptides such as glycosylated peptides. A "variant" in accordance with the present invention is also a peptide which has been modified after collection of the sample, for example by covalent or non-covalent attachment of a label, particularly a radioactive or fluorescent label, to the peptide.

The term "sample" refers to a sample of a body fluid, to a sample of separated cells or to a sample from a tissue or an organ. Samples of body fluids can be obtained by well known techniques and include, preferably, samples of blood, plasma, serum, or urine. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce natriuretic peptides, such as heart tissue, vascular tissues, such as endothelian tissue or tissue of the nervous system .

Comparing as used herein encompasses comparing the amount of the natriuretic peptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically predict whether a subject is at risk of developing cardiovascular complications when suffering already from a bone mineral disorder.

The term "reference amount" as used herein refers to an amount which allows assessing whether a subject will develop cardiovascular complications by a comparison as referred to above. Accordingly, the reference may either be derived from a subject suffering from a bone mineral disorder, wherein said subject is known to be at risk for developing cardiovascular complications within the predictive widow (i.e. a subject which has developed such complications) or from a subject suffering from a bone mineral disorder known not to develop such complications within the predictive window (i.e. a subject which has not developed such complications). The reference amount applicable for an individual subject may vary depending on various physiological parameters such as age, gender, or subpopulation. Thus, a suitable reference amount may be determined by the method of the present invention from a reference sample to be analyzed together, i.e. simultaneously or subsequently, with the test sample. If the reference sample is obtained from a subject known to be at risk for developing a cardiovascular complication an amount of the natriuretic peptide in the test sample which is equal or greater than the amount in the reference sample is indicative for being at risk of developing cardiovascular complications. If the reference sample is obtained from a subject known not be at risk for developing cardiovascular complications, an amount of the natriuretic peptide greater than the reference amount is indicative for being at risk of developing cardiovascular complications. Moreover, it has been found that the 97.5^{th} percentil of NT-proBNP plasma concentration in apparently healthy blood donors is 84 pg/ml for males and 146 pg/ml for females. Thus an amount within the range of 80 to 150 pg/ml and, more preferably, an amount of 125 pg/ml, may serve, preferably, as a general reference amount. It is to be understood that the aforementioned amounts may vary due to statistics and errors of measurement. If the amount of the natriuretic peptide is greater than the aforementioned reference amount, the subject will be at risk of developing a cardiovascular complication within the predictive window.

Advantageously, it has been found in accordance with the present invention that natriuretic peptides and, in particular, NT-proBNP, allow to predict whether a subject suffering from a bone mineral disorder such as osteporosis will also develop cardiovascular complications in the future. Accordingly, thanks to the present invention suitable measures may be applied in order to avoid or ameliorate the development of the said cardiovascular complications. Such measures comprise prophylactic medical treatments as well as nutritional diets or a change in the individual lifestyle in order to avoid lifestyle risk factors such as smoking.

As is evident from the above, the following embodiments are preferred embodiments of the method of the present invention. The aforementioned definition and explanations apply accordingly.

Thus, in a preferred embodiment of the method of the present invention, the said reference amount is obtained from a subject known not to be at risk of developing cardiovascular complications.

More preferably, an amount of the natriuretic peptide larger than the reference amount is indicative for being at risk of developing cardiovascular complications.

In another preferred embodiment of the method of the present invention, the said reference amount is obtained from a subject known to be at risk of developing cardiovascular complications.

More preferably, an amount of the natriuretic peptide larger than or equal to the reference amount is indicative for being at risk of developing cardiovascular complications.

In a further more preferred embodiment of the method of the present invention, the said reference amount of the natriuretic peptide in the said sample is within the range of 80 to 150 pg/ml.

More preferably, an amount of the natriuretic peptide larger than the reference amount is indicative for being at risk of developing cardiovascular complications.

In a preferred embodiment of the method of the present invention, the said subject has not yet developed symptoms associated with cardiovascular complications.

In another preferred embodiment of the method of the present invention, the said cardiovascular complication is a cardiovascular disorder and/or cardiovascular disease.

In a further preferred embodiment of the method of the present invention, the said subject is a female. More preferably, the female is in a postmenopausal stage.

Furthermore, in a preferred embodiment of the method of the present invention, said bone mineral disease or disorder is osteoporosis.

Also in a preferred embodiment of the method of the present invention, said natriuretic peptide is BNP, NT-proBNP or a variant thereof.

The present invention further relates to a device for predicting whether a subject suffering from bone mineral disease or disorder is at risk of developing cardiovascular complications comprising means for determining the amount of a natriuretic peptide in a sample of said subject and means for comparing said amount to a reference amount, whereby it is predicted whether a subject is at risk of developing cardiovascular complications.

The term "device" as used herein relates to a system of means comprising at least the aforementioned means operatively linked to each other as to allow the prediction. Preferred means for determining the amount of natriuretic peptide and for predicting whether a subject is at risk of developing a cardiovascular complication are disclosed above in connection with the method of the invention. How to link the means in an operating manner will depend on the type of means included into the device. For example, where means for automatically determining the amount of the natriuretic peptide are applied, the data obtained by said automatically operating means can be processed by, e.g., a computer program in order to predict whether the subject is at risk of developing a cardiovascular complication. Preferably, the means are comprised by a single device in such a case. Said device may accordingly include an analyzing unit for the measurement of the amount of the natriuretic peptide in an applied sample and a computer unit for processing the resulting data for the prediction. Alternatively, where means such as test stripes are used for determining the amount of the natriuretic peptide, the means for predicting may comprise control stripes or tables allocating the determined amount to an amount known to be accompanied with mild or moderate heart failure or an amount known to be indicative for a healthy subject as discussed above. The test stripes are, preferably, coupled to a ligand which specifically binds to the natriuretic peptide. The strip or device, preferably, comprises means for detection of natriuretic peptide binding to the said ligand. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of diagnostic raw data which need interpretation by the clinician. Preferably, the output of the device are, however, processed diagnostic raw data the interpretation of which does not require a clinician, i.e. it should be inevitably clear from the output whether the subject suffers from mild or moderate heart failure. Further preferred devices comprise the analyzing units/devices (e.g., biosensors, arrays, solid supports coupled to ligands specifically recognizing the natriuretic peptide, Plasmon surface resonace devices, NMR spectrometers, mass- spectrometers etc.) or evaluation units/devices referred to above in accordance with the method of the invention.

Finally, the present invention encompasses a kit for carrying out any one of the aforementioned methods of the present invention, wherein said kit comprises means for determining the amount of a natriuretic peptide in a sample and means for comparing the amount to a reference amount, whereby it is predicted whether the subject is at risk of developing cardiovascular complications.

The term "kit" as used herein refers to a collection of the aforementioned means, preferably, provided in separately or within a single container. The container, also preferably, comprises instructions for carrying out the method of the present invention.

All references cited in this specification are herewith incorporated by reference with respect to their entire disclosure content and the disclosure content specifically mentioned in this specification.

The following Example shall merely illustrate the invention. It shall not be use, whatsoever, to limit the scope of the present invention.

### Example: Case studies relating to postmenopausal females

In three postmanopausal females, 52, 55 and 59 years old with comparabel atheroscelerotic risk (PROCAM score 42, 40 and 38) and without clinical signs of coronary heart disease or heart failure the risk of developing cardiovascular complications or acute cardiovascular events should be investigated.

As could be seen from table 1 two patients were classified as "low bone mass" with total hip BMD Tscore > -2.5 (patient 2 and 3)and one patient was classified as "osteoporosis" with total hip BMD Tscore < -2.5 and prior vertebral fracture (patient 1) indicating that this patient is belonging to a population with a statistically higher prevalence of bone metabolism derived cardiovascular risk.

NT-proBNP determination revealed NT-proBNP concentrations of 120 pg/ml in patient 1 and 83 pg/ml in patient 3 indicating no elevated risk of developing cardiovascular complications or acute cardiovascular events whereas for patient 2 a NT-proBNP concentration of 185 pg/ml was measured indicating an elevated cardiovascular risk. This patient needs further cardiological diagnostic investigation and probably pre-emptive cardiological medication.

The results of the case studies are shown in the following Table 1

**Table 1: Risk stratification by NT-proBNP in Patients with bone metabolism derived cardiovascular risk**

| | Patient 1 | Patient 2 | Patient 3 |
|---|---|---|---|
| **Demographics** | | | |
| Age (years) | 55 | 59 | 52 |
| BMI (Kg/cm² ) | 24 | 26 | 23 |
| Current smoking | no | no | yes |
| Syst. blood pressure (mmHg) | 155 | 150 | 140 |
| diast. blood pressure (mmHG) | 95 | 80 | 85 |
| Hypertension | yes | no | no |
| **Bone metabolism** | | | |
| Total hip BMD Tscore (SD) | -2,8 | -2,3 | -2,0 |
| Prior vertebral fracture | yes | no | no |
| severe Osteoporosis | yes | no | no |
| low bone mass | yes | yes | yes |

| **Atherosclerotic risk factors** | | | |
|---|---|---|---|
| Total Cholesterol (mg/dl) | 240 | 220 | 190 |
| LDL-Chol (mg/dl) | 155 | 140 | 130 |
| HDL-Chol (mg/dl) | 55 | 65 | 70 |
| Triglycerides (mg/dl) | 145 | 150 | 140 |
| CRP (mg/dl) | 1,5 | 1,8 | 1,3 |
| PROCAM-Scare | 40 | 38 | 42 |

| **Additional cardiovasc. criteria** | | | |
|---|---|---|---|
| Fibrinogen (mg/dl) | 270 | 240 | 210 |
| Glucose (fasting) (mg/dl) | 110 | 95 | 90 |
| Diabetes | no | no | no |
| prior MI | no | no | no |
| prio CABG | no | no | no |
| prior stoke | no | no | no |
| signs for heart failure | no | no | no |

| **Medication** | | | |
|---|---|---|---|
| lipid lowering medication | no | no | yes |
| Diuretics | no | no | no |
| ACE inhibitors | no | no | no |
| Calcium channnel blockes | yes | no | no |
| Nitrates | no | no | no |
| Coronary heart disease | no | no | no |
| prior hormone therapy | yes | no | yes |
| Bone metabolism derived cardiovascular risk | high | high | Moderate |
| **NT-proBNP (pg/ml)** | **120** | **185** | **83** |
| **Elevated individual cardiovascular risk** | **no** | **yes** | **No** |

## Claims

1. A method of predicting whether a subject suffering from a bone mineral disorder is at risk of developing cardiovascular complications comprising:
(a) determining the amount of a natriuretic peptide in a sample of said subject; and
(b) predicting whether the subject is at risk of developing cardiovascular complications by comparing the amount determined in step a) to a reference amount.

2. The method of claim 1, wherein said reference amount is obtained from a subject known not to be at risk of developing cardiovascular complications.

3. The method of claim 2, wherein an amount of the natriuretic peptide larger than the reference amount is indicative for being at risk of developing cardiovascular complications.

4. The method of claim 1, wherein said reference amount is obtained from a subject known to be at risk of developing cardiovascular complications.

5. The method of claim 4, wherein an amount of the natriuretic peptide larger than or equal to the reference amount is indicative for being at risk of developing cardiovascular complications.

6. The method of claim 1, wherein said reference amount of the natriuretic peptide in the said sample is within the range of 80 to 150 pg/ml.

7. The method of claim 6, wherein an amount of the natriuretic peptide larger than the reference amount is indicative for being at risk of developing cardiovascular complications.

8. The method of any one of claims 1 to 7, wherein said subject has not yet developed symptoms associated with cardiovascular complications.

9. The method of any one of claims 1 to 8, wherein said cardiovascular complication is a cardiovascular disorder and/or cardiovascular disease.

10. The method of any one of claims 1 to 9, wherein said subject is a female.

11. The method of claim 10, wherein the female is in a postmenopausal stage.

12. The method of any one of claims 1 to 11, wherein said bone mineral disorder is osteoporosis.

13. The method of any one of claims 1 to 12, wherein said natriuretic peptide is BNP, NT-proBNP or a variant thereof.

14. A device for predicting whether a subject suffering from a bone mineral disorder is at risk of developing cardiovascular complications comprising means for determining the amount of a natriuretic peptide in a sample of said subject and means for comparing said amount to a reference amount, whereby it is predicted whether a subject is at risk of developing cardiovascular complications.

15. Kit for carrying out the method of any one of claims 1 to 13 comprising means for determining the amount of a natriuretic peptide in a sample and means for comparing the amount to a reference amount, whereby it is predicted whether the subject is at risk of developing cardiovascular complications.
